Europäisches Patentamt

European Patent Office

Office européen des brevets

(11) Numéro de publication: **0 509 187 A1**

## DEMANDE DE BREVET EUROPEEN

(21) Numéro de dépôt: **91870064.2**

(22) Date de dépôt: **18.04.91**

(51) Int. Cl.5: **G01G 17/02**, G01G 9/00

(43) Date de publication de la demande:
**21.10.92 Bulletin 92/43**

(84) Etats contractants désignés:
**AT BE CH DE DK ES FR GB GR IT LI LU NL SE**

(71) Demandeur: **BARCO AUTOMATION, NAAMLOZE VENNOOTSCHAP**
**Frankrijklaan 18**
**B-8970 Poperinge(BE)**

(72) Inventeur: **De Clercq, André**
**Stockrey Straat, 21**
**B-8520 Kuurne(BE)**

(74) Mandataire: **Bossard, Franz**
**BOSSARD CONSULTANTS S.P.R.L. 288, rue de Jamioulx**
**B-6110 Montigny-le-Tilleul(BE)**

(54) **Procédé de mesure de la masse d'un produit non rigide défilant dans un appareil de mesure.**

(57) Un procédé de mesure de la masse d'un produit non rigide défilant dans un appareil de mesure dans lequel on fait défiler le produit à mesurer à travers un système vibratoire (1) susceptible de résonner à une fréquence propre, déterminée par les dimensions, masses et nature du support du système vibratoire (1) vide, dans lequel on excite cette fréquence propre au moyen d'une source vibratoire (2) appropriée, dans lequel on mesure l'intensité de la vibration au moyen d'un détecteur de vibrations (3), et dans lequel on compare l'intensité mesurée dans le système vibratoire chargé par le produit y défilant avec celle mesurée dans le système vibratoire vide pour en déduire le poids par unité de longueur du produit y défilant.

fig.1

EP 0 509 187 A1

La présente invention a pour objet un procédé de mesure de la masse par unité de longueur d'un produit, non rigide, défilant dans un appareil de mesure. De tels produits, non rigides sont notamment des mèches à fibres très fines et souples utilisées dans des filatures.

Il est connu d'utiliser des procédés de mesure indirectes de la masse d'un produit par unité de longueur qui déduisent cette masse au moyen de la mesure de caractéristiques telles que les dimensions géométriques, l'absorption de la lumière traversant le produit au défilé la perméabilité à des ondes autres que celles de la lumière etc. Ces procédés de mesure connus peuvent être très sensibles à d'autres propriétés du produit à mesurer que la masse par unité de longueur. De ce fait, elles peuvent donner lieu à des appréciations erronées de la masse. Ceci est le cas, en particulier, pour des matériaux fibreux ou cellulaires d'un contour peu déterminé et pesant moins de 30 g/m, par exemple des mèches ou rubans de fibres.

L'invention a pour but principal un procédé de mesure permettant de déterminer la masse par unité de longueur directement et sans intermédiaire, indépendante de la nature du matériau et sans contact physique direct entre le produit à mesurer et un détecteur de mesure devant lequel le produit défile en continu et à grande vitesse. En particulier, le but de l'invention est la mesure de la masse par unité de longueur de mèches, rubans etc. en matières textiles très souples.

Suivant l'invention, ce procédé est caractérisé en ce qu'on fait défiler le produit à mesurer à travers un système vibratoire susceptible de résonner à une fréquence propre, déterminée par les dimensions, masses et nature du support du système vibratoire vide, en ce qu'on excite cette fréquence propre au moyen d'une source vibratoire appropriée, en ce qu'on mesure l'intensité de la vibration au moyen d'un détecteur de vibrations et en ce qu'on compare l'intensité mesurée dans le système vibratoire chargé par le produit y défilant avec celle mesurée dans le système vibratoire vide pour en déduire le poids par unité de longueur du produit y défilant.

Le terme "système vibratoire vide" doit être compris dans un sens large qui inclut un vide relatif, par exemple un système comprenant un produit de 2g/m, si le produit à mesurer pèse en moyenne 10 g/m.

L'invention concerne aussi un appareil de mesure convenant au procédé décrit, ainsi qu'une machine textile utilisant ce procédé.

L'invention est expliquée ci-dessous par rapport à quelques exemples d'appareils convenant à l'exécution du procédé, et à des exemples de résultats de mesure, en se référant au dessin annexé. La figure 1 de ce dessin est une coupe à travers un appareil de mesure. La figure 2 est un diagramme montrant des résultats de mesure, et les figures 3 et 4 représentent des variantes d'appareils de mesure.

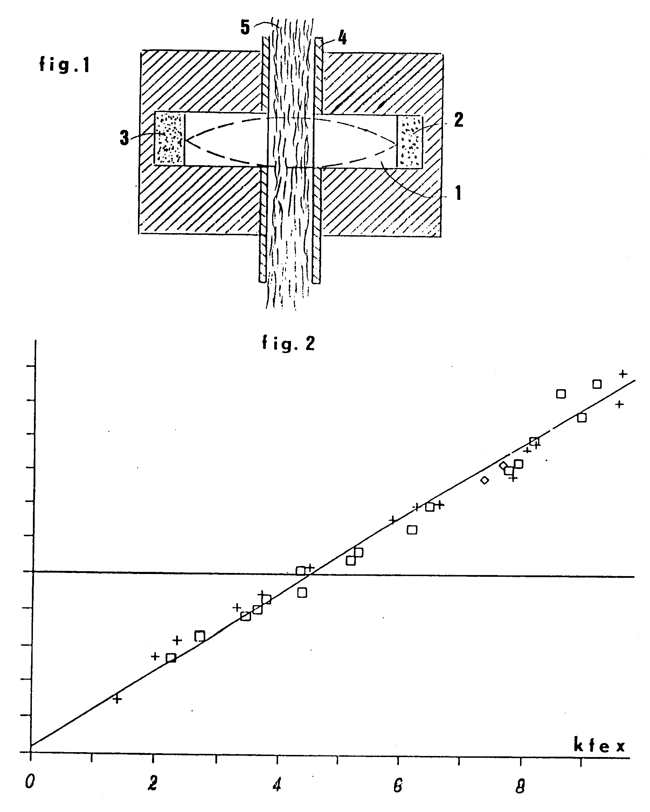

Dans l'exemple représenté à la figure 1, le système vibratoire est un résonateur acoustique 1, cylindrique (il peut aussi être de section carrée ou autre), constitué par une cavité de longueur L et de largeur ou diamètre D. Il est équipé d'une source acoustique 2 placée à une des extrémités et d'un récepteur acoustique 3 placé à l'extrémité opposée de la cavité. La source 2 est par exemple un haut-parleur; le récepteur 3 peut être un microphone. Le haut-parleur est réglable pour émettre une fréquence déterminée, à savoir une fréquence propre, de préférence la fréquence propre, fondamentale du résonateur pour laquelle l'intensité acoustique mesurée à l'endroit du microphone est maximale.Cependant, il n'est pas nécessaire que ce haut-parleur émette une fréquence absolument pure. En effet, dans un résonateur, seule les fréquences propres sont amplifiées; toute autre fréquence est étouffée.

Au milieu, la cavité du résonateur est traversé par un conduit 4, qui véhicule le produit à mesurer, en l'occurrence une mèche 5 en fibres textiles. De préférence, le diamètre du conduit 4 est égal à la largeur du résonateur. L'identité de ces deux dimensions peut présenter certains intérêts pratiques, mais n'est pas nécessaire. De préférence, il n'existe pas de séparation entre la cavité du résonateur et celle du conduit 4. Il est possible de séparer le canal 4 de la cavité du résonateur au moyen d'une membrane en matière très légère et souple, transparente à l'onde vibratoire et résistante à l'abrasion par le produit qui défile à grande vitesse dans son intérieur. De telles membranes diminuent cependant la sensibilité du procédé de mesure, mais ont l'avantage d'éviter ou au moins diminuer l'encrassement de l'appareil de mesure.

Le produit à mesurer peut défiler à très grande vitesse à travers le résonateur, grâce à des dispositifs d'entraînement, non représentés.

Comme il n'existe pas de séparation entre la cavité du résonateur et celle du conduit 4, il est utile ou même nécessaire, dans la plupart des cas, de prévoir des moyens de nettoyage du système vibratoire, en l'occurrence de la cavité résonnante. Ces moyens peuvent être intermittents (brossage, soufflage) ou continus (courants d'air balayant continuellement la cavité du résonateur au départ du haut-parleur 2 et du microphone 3 vers le canal 4 et l'extérieur).

Le matériau entourant la cavité du résonateur 1 est un matériau possédant un coefficient d'amortissement élevé pour des vibrations, notamment pour des ondes acoustiques, par exemple du caoutchouc de moulage ou de la mousse de polyuréthanne. On évite ainsi que les ondes acoustiques prennent leur chemin en partie par le matériau entourant la cavité du résonateur et perturbent des mesures.

Les dimensions de cette cavité sont choisies en fonction des mesures à effectuer. Par exemple, une mèche de section maximuale de 1,3 cm² nécessite un conduit 4 de même section. La cavité du résonateur peut présenter alors aussi la même largeur. Dans ce cas, les particules d'une mèche défilant à grande vitesse à travers le résonateur, par exemple à 16 m/s restent à l'intérieur du résonateur pendant 0,8 ms. Il est hautement souhaitable que pendant ce temps les particules de la mèche subissent au moins 3 vibrations complètes à l'intérieur du résonateur. Dans ce cas, et en supposant une section carrée de la cavité, la demi-période de l'oscillation à la fréquence propre, fondamentale du résonateur doit être égale ou inférieure à 0,13 ms ou, ce qui revient au même, la fréquence de l'oscillation à l'intérieur du résonateur doit être égale ou supérieure à 3,8 kHz. Dans un tel cas, la longueur de la cavité peut être calculée suivant la formule:

$$L = c/(2f),$$

où c = la vitesse du son dans l'air, et
f = la fréquence propre, fondamentale du résonateur,
soit: L = 44 mm.

Si on désire diminuer la fréquence, la section de la cavité du résonateur doit être élargie, et sa longueur augmentée. Si le procédé est utilisé dans des locaux, par exemple d'une filature, il n'est pas souhaitable de choisir un résonateur avec une fréquence propre, fondamentale trop basse, car les vibrations provenant de machines dans une filature peuvent atteindre des valeurs aux environs de 500 Hz. Ceci pourrait perturber des mesures qui s'effectueraient à des fréquences en dessous de 1000 Hz. D'un autre côté, dans le but de garantir une bonne résolution de la grandeur mesurée, une cavité résonnante doit posséder, de préférence, une longueur égale à au moins trois fois la largeur du conduit 4. Ceci impose une limite supérieure à la fréquence propre, fondamentale d'un résonateur.

Le titre de la mèche en ktex (kg/km ou g/m), défilant dans le conduit 4, peut être déduit d'une mesure d'intensité à l'endroit du détecteur 3, d'une part lorsque le résonateur est vide, et d'autre part lorsque le résonateur est traversé par le produit qui y défile à grande vitesse. La mesure ou le calibrage à résonateur vide ne change pas ou seulement très lentement. Elle peut donc être mémorisée pour des périodes plus ou moins longues.

Les changements du calibrage à résonateur vide peuvent être dûs, notamment à l'encrassement de la cavité résonnante, et à la variation de la température, de la pression barométrique, et de l'humidité ambiantes. Si seulement des changements de l'atmosphère ambiante sont à craindre, un résonateur de référence à proximité du résonateur de mesure peut être utilisé. Il est possible aussi de mesurer la température, la pression barométrique et l'humidité ambiantes et d'apporter les corrections nécessaires en fonction de ces variables suivant des courbes d'étalonnage, par exemple.

Si au contraire, le résonateur de mesure même doit être calibré à intervalles plus ou moins réguliers, le canal 4 peut être disposé dans une pièce mobile, non représentée, comprenant aussi un canal vide. Par déplacements temporaires de cette pièce mobile, le canal 4 dans lequel défile le produit à mesurer est sorti de la cavité du résonateur et y est remplacé par le canal vide. Ainsi il est possible d'effectuer des calibrages à résonateur vide, sans interruption de l'alimentation d'un dispositif d'étirage.

La mesure de l'intensité, au moyen du microphone 3, lors du défilement du produit dans le canal 4, peut être affichée directement, ou de préférence après avoir été convertie en mesure du titre instantané au moyen d'une courbe d'étalonnage ou grâce à un calcul simple.

Ce calcul peut faire usage de la formule $T^2 = Y (Z^2/X^2 - 1)$, dans laquelle T est le titre, Y un facteur de calibrage, Z l'intensité mesurée, résonateur vide, et X l'intensité mesurée, résonateur chargé du produit y défilant. Le facteur Y ne dépend pas de l'intensité émise par la source acoustique 2; il peut cependant varier un peu en fonction des caractéristiques de l'air (température, pression, humidité), mais ces variations ne sont pas sensibles dans des conditions de fonctionnement normales, ou peuvent être compensées à l'aide d'un dispositif de référence.

Dans le procédé suivant l'invention, le produit à mesurer, c'est-à-dire des fibres très fines et très souples, est supporté par l'air dont est rempli la cavité du résonateur ou un autre fluide. cet air ne change pas d'un produit à mesurer à l'autre. Il suffit de veiller à ne pas introduire un autre support, ce qui arrive si le produit à mesurer est, par exemple, un fil relativement rigide.

Lorsque le produit à mesurer ne présente pas tout à fait les caractéristiques idéales à l'application du procédé suivant l'invention, par exemple si les fibres sont relativement longues ou de diamètre plus important, le procédé suivant l'invention est encore applicable, mais il sera nécessaire de procéder à des étalonnages pour chaque produit. En effet, en ce moment, le module de flexion du matériau perturbe un peu la mesure.

Le diagramme, figure 2 montre en ordonnée une valeur calculée à partir de l'intensité mesurée, résonateur chargé du produit y défilant, et en abscisse, le titre de mèches de nature différente: polyester (carrés), coton (croix) et mélanges de polyester et coton (losanges). Il apparaît clairement que la nature de la mèche n'influence pas le diagramme, seule est importante la masse par unité de longueur.

Il est également possible de se libérer complètement de la valeur de l'intensité mesurée à l'endroit du détecteur 3. Dans ce cas, la mesure de cette intensité sert uniquement pour déterminer exactement la fréquence propre fondamentale du résonateur pour laquelle doit être réglée la source acoustique 2. C'est la fréquence pour laquelle l'intensité mesurée à l'endroit du microphone est maximale. On détermine donc comme auparavant la fréquence propre fondamentale du résonateur vide et on la mémorise. Ensuite, on détermine la fréquence propre fondamentale du résonateur chargé du produit y défilant en variant la fréquence émise par la source acoustique jusqu'à ce que l'intensité mesurée par le microphone est maximale. En connaissant les deux fréquences propres fondamentales, la masse du produit y défilant peut être déduite grâce à une courbe d'étalonnage ou grâce à une formule mathématique. Ce deuxième procédé nécessite un peu plus de temps, mais sa précision est excellente.

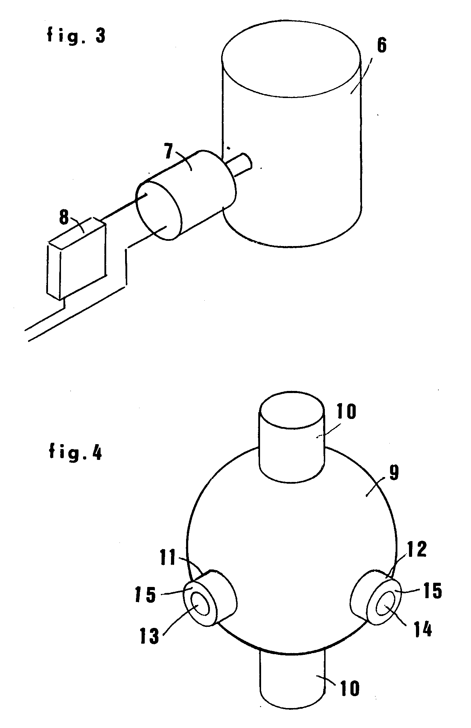

D'autres systèmes vibratoires que celui décrit ci-dessus peuvent être envisagés comme le montrent les exemples de variantes représentés aux figures 3 et 4.

A la figure 3, le système vibratoire est un tronçon de canal 6 dans lequel est véhiculé le produit à mesurer. Ce tronçon 6 est relié à une source vibratoire électromagnétique 7 alimentée au moyen d'un courant alternatif à fréquence variable. Dans les conducteurs d'alimentation de cette source 7 est disposé un appareil de mesure d'impédance électrique 8 de la source vibratoire 7. La fréquence du courant alternatif d'alimentation de la source 7 est réglé pour correspondre à une fréquence propre du système vibratoire, de préférence à la fréquence propre fondamentale de ce système. Lorsque le produit à mesurer défile dans le canal 6, l'impédance mesurée dans l'appareil 8 devient d'autant plus grande que la fréquence propre du système vibratoire chargé s'écarte de la fréquence propre du système vibratoire vide. Cette impédance peut être affichée telle quelle ou de préférence après conversion en titre de la mèche à mesurer.

A la figure 4, le système vibratoire est un résonateur sphérique. Il comprend une sphère résonnante 9, deux bouts d'un canal 10 pour véhiculer le produit à mesurer à travers le résonateur, et deux ouvertures avec embouts 11 et 12. Dans ces ouvertures avec embouts 11 et 12, sont logés, respectivement une source vibratoire 13 et un détecteur de vibrations 14. La source 13 et le détecteur 14 sont enveloppés dans des manteaux 15 en matière absorbante pour les vibrations.

Le procédé décrit est applicable non seulement dans l'industrie textile , mais aussi, par exemple dans l'industrie chimique. Il peut permettre le dosage de poudres, de gaz ou de liquides, chargés ou non. Par contre, le procédé suivant l'invention n'est pas utilisable pour des produits qui agissent comme réflecteur pour les vibrations, en l'occurrence les ondes acoustiques. C'est pour cette raison que les produits rigides ne peuvent pas être mesurés par le procédé suivant l'invention.

L'invention peut être utilisée aussi pour la mesure indirecte d'autres propriétés que la masse, mais dont la masse constitue un paramètre essentiel.

**Revendications**

1. Procédé de mesure de la masse d'un produit non rigide défilant dans un appareil de mesure approprié,

   caractérisé en ce qu'on fait défiler le produit à mesurer à travers un système vibratoire (1) susceptible de résonner à une fréquence propre, déterminée par les dimensions, masses, et nature du support du système vibratoire (1) vide, en ce qu'on excite cette fréquence propre au moyen d'une source vibratoire (2) appropriée, en ce qu'on mesure l'intensité de la vibration au moyen d'un détecteur de vibrations (3), et en ce qu'on compare l'intensité mesurée dans le système vibratoire chargé par le produit y défilant avec celle mesurée dans le système vibratoire vide pour en déduire le poids par unité de longueur du produit y défilant.

2. Procédé de mesure de la masse d'un produit non rigide défilant dans un appareil de mesure approprié,

   caractérisé en ce qu'on fait défiler le produit à mesurer à travers un système vibratoire (1), susceptible de résonner à une fréquence propre, déterminée par les dimensions du système vibratoire vide, en ce qu'on excite cette fréquence propre au moyen d'une source de vibrations (2) appropriée, en ce qu'on mesure l'intensité des vibrations au moyen d'un détecteur de vibrations (3), et en ce qu'on détermine la fréquence propre fondamentale du système vibratoire chargé par le produit y défilant, et qu'on la compare avec celle mesurée dans le système vibratoire vide pour en déduire le poids par unité de longueur du produit y défilant.

3. Procédé suivant une des revendications précédentes, caractérisé en ce que le détecteur de vibrations est remplacé par un dispositif de mesure de l'impédance électrique de la source vibratoire, ce dispositif de mesure d'impédance étant placé dans le circuit électrique d'alimentation de la source vibratoire.

4. Procédé suivant une quelconque des revendications précédentes, caractérisé en ce que le temps de passage d'une particule du produit défilant à travers le système vibratoire (1) est choisi égal à au moins 6 fois la demi-période de la fréquence propre fondamentale du système vibratoire vide.

5. Procédé suivant une quelconque des revendications précédentes, caractérisé en ce que le système vibratoire est un résonateur (1).

6. Procédé suivant la revendication 5, caractérisé en ce qu'on mène le produit, grâce à un conduit (4), à travers le ventre de l'oscillation au milieu de la cavité du résonateur (1).

7. Procédé suivant une des revendications 5 ou 6, caractérisé en ce que la longueur de la cavité du résonateur (1) est au moins égale à trois fois la largeur du conduit (4) dans lequel défile le produit à mesurer.

8. Appareil de mesure pour l'application du procédé suivant l'une quelconque des revendications précédentes, caractérisé en ce que le système vibratoire est supporté ou entouré par un matériau possédant un coefficient d'amortissement élevé pour des vibrations.

9. Appareil suivant la revendication 8, caractérisé en ce qu'il comporte des moyens de nettoyage du système vibratoire.

10. Appareil suivant une quelconque des revendications 8 ou 9, caractérisé en ce que le canal (4) conduisant le produit à mesurer se trouve dans une pièce mobile comprenant aussi un canal vide, cette pièce mobile étant déplacée temporairement pour que le canal vide se mette à la place du canal (4) véhiculant le produit à mesurer.

11. Machine textile, caractérisée en ce qu'elle utilise le procédé de mesure suivant une quelconque des revendications 1 à 7.

fig.1

fig. 2

fig. 3

fig. 4

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| X | JAPANESE JOURNAL OF APPLIED PHYSICS. vol. 26, no. 11, Novembre 1987, PART 1,TOKYO JP pages 1395 - 1396; SHIGERU NAGAYAMA: 'Microwave sensor of basis weight and moisture of sheet materials by re-entrant cavity' * page 1396, colonne de gauche, ligne 25 - ligne 35; figure 1 * | 1,2,5,6 | G01G17/02 G01G9/00 |
| Y | --- | 8,11 | |
| Y | US-A-3 470 734 (N.B. AGDUR ET AL) * colonne 2, ligne 2 - ligne 22; figure 1 * --- | 8 | |
| Y | US-A-3 486 369 (A.G.K. KORZILIUS) * colonne 1, ligne 28 - ligne 30 * * colonne 6, ligne 64 - ligne 75; figure 1 * --- | 11 | |
| A | GB-A-2 089 983 (GAO GESELLSCHAFT FÜR AUTOMATION UND ORGANISATION MBH) * abrégé * * page 3, ligne 71 - ligne 73; figure 1 * --- | 1,2 | |
| A | EP-A-0 292 571 (DIPOLE ELECTRONICS CO. LTD.) * page 12, ligne 14 - ligne 21; figure 5A * --- | 1,2,8 | **DOMAINES TECHNIQUES RECHERCHES (Int. Cl.5)** G01G G01N |
| A | CH-A-366 686 (H.L. USTER) * revendication 1 * ----- | 1,2,8,11 | |

Le présent rapport a été établi pour toutes les revendications

| Lieu de la recherche | Date d'achèvement de la recherche | Examinateur |
|---|---|---|
| LA HAYE | 04 DECEMBRE 1991 | GANCI P.A. |

EPO FORM 1503 03.82 (P0402)